# EUROPEAN PATENT APPLICATION

(11) **EP 1 391 200 A1**
(43) Date of publication of application: **25.02.2004**
(21) Application number: 02726486.0
(22) Date of filing: 24.05.2002
(51) Int. Cl.: A61K 31/198, A61K 9/20, A61K 31/439, A61K 45/00, A61K 47/02, A61K 47/12, A61K 47/36, A61P 11/10, A61P 11/14

(54) **DRUG PREPARATIONS**

(30) Priority: 25.05.2001 JP 2001156853
(71) Applicant: SSP Co., Ltd., Chuo-ku, Tokyo 103-8481 (JP)
(72) Inventor: ONUKI, Yoichi, Narita-shi, Chiba 286-0015 (JP); OKADA, Minoru, Inzai-shi, Chiba 270-1323 (JP); SAKAI, Hirotaka, Yotsukaido-shi, Chiba 284-0005 (JP); KANBE, Hideyoshi, Ichikawa-shi, Chiba 272-0826 (JP); MIZUNO, Hiroyuki, Inba-gun, Chiba 286-0201 (JP); IMAMORI, Katsumi, Yotsukaido-shi, Chiba 284-0006 (JP)
(74) Representative: Hartz, Nikolai
(86) International application number: PCT/JP2002/005041
(87) International publication number: WO 2002/096405

(57) **Abstract**

Drug preparations characterized by containing fudosteine and an antitussive. These preparations are drugs to be used for common cold, etc. which have improved antitussive and sputum-removal effects.

## Description

### TECHNICAL FIELD

The present invention relates to a drug preparation, more particularly, to a drug preparation that has antitussive and expectorant effects and can be advantageously used as an antitussive expectorant or the like.

### BACKGROUND ART

Conventionally, an antitussive component and expectorant component are added to many drug preparations for colds, since diseases such as the common cold cause coughs and sputum. Many antitussive expectorants that mainly aim to suppress coughs and remove sputum are also commercially available.

However, the commercially available drug preparations for colds are not sufficiently effective in suppressing coughs and removing sputum, since the amount of the antitussive and expectorant components is limited. Neither are the commercially available antitussive expectorants sufficiently effective, particularly in the antitussive effect.

Therefore, development of drug preparations having an improved antitussive effect has been desired.

### DISCLOSURE OF THE INVENTION

In order to obtain drug preparations having improved antitussive and expectorant effects, the present inventors have studied the efficacy of various antitussives and their combinations with other medicinal components and have found that if fudosteine, which is useful as an expectorant but does not exhibit an antitussive effect by itself, is combined with an antitussive, the antitussive can exhibit a markedly enhanced antitussive effect. This finding has led to the completion of the present invention.

Specifically, the present invention provides drug preparations containing fudosteine and an antitussive.

### BEST MODE FOR CARRYING OUT THE INVENTION

Fudosteine and an antitussive are added to the drug preparation of the present invention as essential medicinal components.

The fudosteine used in the present invention is S-(3-hydroxypropyl)-L-cysteine of the following formula (I) that has been already reported to be useful as an expectorant in Japanese Patent Application Laid-open No. 2-3674.

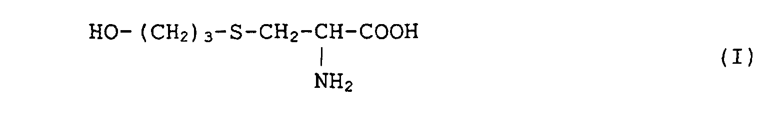

However, a drug preparation to which the fudosteine and an antitussive are added has not yet been known.

In the drug composition of the present invention, fudosteine or its salt such as an inorganic acid salt, organic acid salt, alkaline metal salt, or alkaline earth metal salt of fudosteine can be used.

Although there are no specific limitations to the amount of fudosteine or its salt in the drug composition of the present invention, a dose for an adult is usually about 12-2, 400 mg/day, and more preferably about 40-1,200 mg/day.

Examples of the antitussive used in the drug preparation of the present invention include alloclamide hydrochloride, cloperastine hydrochloride, pentoxyverine citrate, tipepidine citrate, sodium dibunate, dextromethorphan hydrobromide, dextromethorphan phenolphthalin salt, tipepidine hibenzate, cloperastine fendizoate, codeine phosphate, dihydrocodeine phosphate, trimetoquinol hydrochloride, phenylpropanolamine hydrochloride, methoxyphenamine hydrochloride, dl-methylephedrine hydrochloride, 1-methylephedrine hydrochloride, noscapine hydrochloride, noscapine, ephedra herb, nandina fruit, dl-methylephedrine saccharin salt, carbetapentane citrate, dimemorfan phosphate, benproperine phosphate, isoaminile citrate, oxeladin citrate, oxeladin tannate, eprazinone hydrochloride, clobutinol hydrochloride, clofedanol hydrochloride, fominoben hydrochloride, cloperastine hydrochloride, oxymethebanol, opium, ethylmorphine hydrochloride, morphine hydrochloride, morphine sulfate, opium alkaloids hydrochlorides, TRK-851, and CS-003. These can be used either individually or in combination of two or more.

The amount of the antitussive in the drug preparation of the present invention is determined based on the type and amount of other medicines used in combination according to a known formulation technique. A dose for an adult is usually 1 mg to 20 g/day, and preferably 1.2 mg to 10 g/day.

Besides the above essential medicinal components, other medicinal components may be added to the drug preparation of the present invention. For example, one or more medicinal components selected from the group consisting of a bronchodilator, antihistamine, analeptic, galenical, antacid or mucosal protective agent, and bactericide may be added.

Examples of the bronchodilator include aminophylline, diprophylline, theophylline, proxyphylline, choline theophylline, epinephrine, ephedrine hydrochloride, isoprenaline sulfate, isoprenaline hydrochloride, orciprenaline sulfate, clorprenaline hydrochloride, salbutamol sulfate, terbutaline sulfate, tulobuterol hydrochloride, procaterol hydrochloride, fenoterol hydrobromide, formoterol fumarate, clenbuterol hydrochloride, mabuterol hydrochloride, ipratropium bromide, flutropium bromide, oxitropium bromide, tiotropium bromide, salmeterol xinafoate, hexoprenaline sulfate, Ro 25-1553, and a vasoactive intestinal peptide/pituitary adenylate cyclase activating polypeptide derivative (VIP/PACAP derivative). These can be used either individually or in combination of two or more.

The amount of the bronchodilator is determined based on the type and amount of other medicines used in combination according to a known formulation technique. A dose for an adult is usually 10 µg to 1, 000 mg/day, and preferably 42-600 mg/day.

Examples of the antihistamine include isothipendyl hydrochloride, iproheptine hydrochloride, difeterol hydrochloride, diphenylpyraline hydrochloride, diphenhydramine hydrochloride, triprolidine hydrochloride, tripelennamine hydrochloride, thonzylamine hydrochloride, fenethazine hydrochloride, promethazine hydrochloride, diphenhydramine salicylate, carbinoxamine diphenyldisulfonate, alimemazine tartrate, diphenhydramine tannate, diphenylpyraline theoclate, promethazine methylene disalicylate, carbinoxamine maleate, dl-chlorpheniramine maleate, d-chlorpheniramine maleate, difeterol phosphate, methdilazine hydrochloride, mebhydroline napadisylate, mequitazine, cyproheptadine hydrochloride, clemastine fumarate, epinastine hydrochloride, olopatadine hydrochloride, fexofenadine hydrochloride, loratadine, bepotastinebesilate, mizolastine, and NIP-531. These can be used either individually or in combination of two or more.

The amount of the antihistamine is determined based on the type and amount of other medicines used in combination according to a known formulation technique. A dose for an adult is usually 1-300 mg/day, and preferably 3-150 mg/day.

Examples of the analeptic include caffeine and sodium benzoate, caffeine, anhydrous caffeine, dl-methylephedrine hydrochloride, dl-methylephedrine saccharin salt, ephedrine hydrochloride, phenylpropanolamine hydrochloride, phenylephrine, 1-methylephedrine hydrochloride, methoxyphenamine hydrochloride, dl-epinephrine hydrochloride, dl-isoproterenol hydrochloride, isoproterenol sulfate, orciprenaline sulfate, terbutaline sulfate, salbutamol sulfate, trimethoquinol hydrochloride, hexoprenaline sulfate, clorprenaline hydrochloride, tulobuterol hydrochloride, procaterol hydrochloride, pirbuterol hydrochloride, fenoterol hydrobromide, formoterol fumarate, clenbuterol hydrochloride, mabuterol hydrochloride, ethylcysteine hydrochloride, methylcysteine hydrochloride, and pseudoephedrine. These can be used either individually or in combination of two or more.

The amount of the analeptic is determined based on the type and amount of other medicines used in combination according to a known formulation technique. A dose for an adult is usually 1-900 mg/day, and preferably 5-300 mg/day.

Examples of the galenical include powders of galenicals such as ephedra herb, nandina fruit, Japanese cherry bark, polygala root, glycyrrhiza, platycodon root, apricot kernel, plantago seed, plantago herb, lycoris, senega, kudzu vine root, fritillary bulb, gambir, fennel, scutellaria root, trichosanthes seed, cinnamon bark, oriental bezoar, schisandra fruit, asiasarum root, tatarian aster root, musk, codonopsis root, ginger, mulberry bark, perilla herb, panax rhizome, citrus unshiu peel, ginseng, ophiopogon tuber, and pinellia tuber, and their extracts. These can be used either individually or in combination of two or more.

The amount of the galenical is determined based on the type and amount of other medicines used in combination according to a known formulation technique. A dose for an adult is usually 0.001-300 g/day (as an extract, converted into the raw galenical) or 0.0004-60 g/day (as a powder), and preferably 0. 005-10 g/day (as an extract, converted into the raw galenical) or 0.001-3 g/day (as a powder).

Examples of the antacid or mucosal protective agent include aminoacetic acid, magnesium oxide, magnesium carbonate, magnesium silicate, synthetic aluminum silicate, synthetic hydrotalcite, dihydroaluminum aminoacetate, aluminum hydroxide gel, aluminum hydroxide dried gel, aluminum hydroxide-magnesium carbonate mixture co-dried gel, aluminum hydroxide-sodium hydrogencarbonate coprecipitate, aluminum hydroxide-calcium carbonate-magnesium carbonate coprecipitate, magnesium hydroxide-aluminum potassium sulfate coprecipitate, magnesium aluminometasilicate, magnesium hydroxide, aluminum sulfate, and sucralfate. These can be used either individually or in combination of two or more.

The amount of the antacid or mucosal protective agent is determined based on the type and amount of other medicines used in combination according to a known formulation technique. A dose for an adult is usually 10-8,000 mg/day, and preferably 100-4,000 mg/day.

Examples of the bactericide include cetylpyridium chloride, dequalinium chloride, and chlorhexidine hydrochloride. These can be used either individually or in combination of two or more.

The amount of the bactericide is determined based on the type and amount of other medicines used in combination according to a known formulation technique. A dose for an adult is usually 0.05-50 mg/day, and preferably 0.125-5 mg/day.

A vitamin and antiinflammatory enzyme may be further added to the present drug preparation.

Examples of the vitamin include vitamin B1 and its derivatives such as octotiamine, prosultiamine, fursultiamine, fursultiamine hydrochloride, bisbentiamine, benfotiamine, dicethiamine hydrochloride, cycotiamine, cocarboxylase, thiamine disulfide, thiamine hydrochloride, thiamine nitrate, bisthiamine nitrate, thiamine dicetylsulfate, and bisibutiamine, salts thereof, vitamin B2 and its derivatives such as riboflavin, riboflavin butyrate, riboflavin sodium phosphate, and flavin adenine dinucleotide sodium, salts thereof, vitamin C and its derivatives such as ascorbic acid, sodium ascorbate, and calcium ascorbate, salts thereof, hesperidin and its derivatives, salts thereof, vitamin F, vitamin A and its derivatives such as retinol acetate and retinol palmitate, salts thereof, vitamin E and its derivatives such as tocopherol succinate, tocopherol calcium succinate, tocopherol acetate, and tocopherol, and salts thereof. These can be used either individually or in combination of two or more.

The amount of the vitamin is determined based on the type and amount of other medicines used in combination according to a known formulation technique. A dose for an adult is usually 0.1-2,000 mg/day, and preferably 1-500 mg/day.

Examples of the antiinflammatory enzyme include bromelain, pronase, serrapeptase, semialkaline proteinase, streptokinase, streptodornase, and lysozyme chloride. These can be used either individually or in combination of two or more.

A dose for an adult of the antiinflammatory enzyme is usually 4-300 mg/day, and preferably 15-60 mg/day.

Expectorants other than fudosteine such as potassium guaiacolsulfonate, guaifenesin, potassium iodide, foeniculated ammonia spirit, sodium hydrogencarbonate, bromhexine hydrochloride, carbocysteine, ambroxol hydrochloride, methylcysteine hydrochloride, acetylcysteine, ethylcysteine hydrochloride, eprazinone hydrochloride, ammonium chloride, potassium cresolsulfonate, l-menthol, trimetoquinol hydrochloride, phenylpropanolamine hydrochloride, and methoxyphenamine hydrochloride, for example, may be added to the drug preparation of the present invention individually or in combination of two or more.

A dose for an adult of these expectorants is usually 1-3,000 mg/day, and preferably 6-900 mg/day.

The drug preparation of the present invention is prepared by appropriately combining the above-described components with a pharmaceutically acceptable additive into a solid, semisolid, or liquid preparation to be orally administered such as a tablet preparation, granule preparation, fine granule preparation, powder preparation, hard capsule preparation, soft capsule preparation, pellet preparation, troche preparation, drop preparation, suspension preparation, emulsion preparation, liquid preparation for internal use, syrup preparation, dry syrup preparation, inhalant preparation, or oral mucoadhesive preparation, according to a conventional method. The medicinal components may be made into microparticles such as microcapsules, nanocapsules, microspheres, or nanospheres prior to preparing the above-mentioned preparation.

There are no specific limitations to the method for preparing the drug preparation of the present invention. Various methods can be employed. For example, when it is necessary to prepare a granulated powder for preparing a tablet preparation, granule preparation, fine granule preparation, powder preparation, capsule preparation, pellet preparation, dry syrup preparation, or the like, conventional granulation is employed. Examples of the granulation include wet granulation using a solution or dispersion liquid containing water or an organic solvent such as spray granulation, agitating granulation, fluidized bed granulation, rolling granulation, or rolling fluidized bed granulation and dry granulation using a powdery binder such as compression granulation.

A powder or granulated powder containing the medicinal components can be mixed and packed into a plurality of packages to prepare a granule preparation, fine granule preparation, or powder preparation. A powder preparation, granulated powder, small tablet preparation, or the like is capsuled using a capsuling machine to prepare a capsule preparation. A powder of the medicinal components, powder preparation, fine granule preparation, granule preparation, or pellet preparation is mixed with an additive and the mixture is compression molded to prepare a tablet preparation.

A tablet preparation, granule preparation, or the like is coated with a coating agent using pan coating, fluidized bed coating, roll coating, or a combination of these, to prepare a coated preparation such as sugar-coated tablets, film-coated tablets, or coated granules. The coating agent may be applied by spray coating wherein the agent is dissolved and/or dispersed in water or an organic solvent or dry coating wherein the agent is directly sprayed and heat, pressure, or the like is applied. The amount of the coating agent can be appropriately selected according to the dosage form or the like. The amount is usually about 0.1-100 weight% for a tablet preparation, about 0.1-200 weight% for a pellet or granule preparation, or about 0.1-300 weight% for a fine granule preparation.

Usually, a liquid preparation for internal use such as a syrup preparation, elixir preparation, limonade preparation, extract preparation, or drink preparation, as well as a hard capsule preparation, soft capsule preparation, or the like in which a liquid or semisolid substance is capsuled, can be produced by a method wherein the medicinal components are mixed with, dissolved in, or dispersed in a part of a solvent such as purified water, followed by adjusting the liquid amount by adding the remaining part of the solvent to the mixture. The pH may be appropriately adjusted using an acid or alkali. The preparation containing a fat-soluble component may be solubilized, emulsified, or suspended using an additive such as a surfactant, solubilizer, emulsifier, or suspending agent. As required, the mixture may be heated, cooled, replaced with nitrogen, filtered, or sterilized during the adjustment.

The drug preparation of the present invention may be appropriately provided with properties such as stability, sustained releasability, durability, or capability of rapid disintegration or rapid dissolution of the medicinal components, improved solubility, taste masking properties, and capability of imparting improved feeling during the dosing, using an additive or the like. A conventional method can be used for providing these properties. Examples of the method include various methods such as a method of adding the medicinal components to separate granules, a method of preparing multilayer granules from the components, a method of preparing multilayer tablets or core tablets from the components, a method of preparing separate granules from the components and tableting them, a method of preparing microcapsules from the components, a method of preparing a coated preparation such as sugar-coated tablets, film-coated tablets, or coated granules from the components, a method of preparing an effervescent preparation from the components, a method of preparing a chewable preparation from the components, a method of preparing an orally disintegrating preparation from the components, a method of preparing a matrix preparation from the components, a method of pulverizing the components all together, a method of preparing a solid solution from the components, a method of adding a sweetener or cooling agent to the components, a method of adding an antioxidant or stabilizer to the components, and a method of adjusting the composition to have a specific pH, viscosity, osmotic pressure, or salt concentration. These methods may be used in combination.

As pharmaceutically acceptable additives used in the drug preparation of the present invention, a stabilizer, surfactant, plasticizer, lubricant, solubilizer, reductant, buffer, sweetener, base, adsorbent, flavor enhancer, binder, suspending agent, antioxidant, brightener, coating agent, humectant, filler, antifoaming agent, cooling agent, colorant, odorant, flavor, sugar-coating agent, isotonizing agent, softener, emulsifier, thickener, effervescent agent, pH adjuster, diluent, and other additives such as an excipient, dispersant, disintegrant, disintegrating assistant, disintegration retardant, perfume, desiccant, antiseptic, preservative, dissolving agent, dissolving assistant, solvent, fluidizer, antistatic agent, extender, moisture retainer, and moisturizer can be given. More specifically, the following additives can be given. These can be used either individually or in combination of two or more.

Specific examples of the excipient, base, or filler include DL-alanine, dl-malic acid, D-sorbitol, D-sorbitol solution, D-mannitol, L-aspartic acid, L-glutamine, β-cyclodextrin, aminoethylsulfonic acid, powdered starch syrup, gum arabic, gum arabic powder, alginic acid, sodium alginate, propylene glycol alginate, pregelatinized starch, inositol, ethanol, ethyl cellulose, octyldecyl triglyceride, opadry beige, olive oil, oleic acid, kaolin, cacao butter, casein, sodium caseinate, carrageenan, caramel, carnauba wax, carboxyvinyl polymer, sodium carboxymethyl starch, carmellose, carmellose calcium, carmellose sodium, glycyrrhiza powder, agar, agar powder, xanthan gum, xylytol, citric acid, sodium citrate, disodium citrate, glycine, glycerol, glycerol fatty acid ester, calcium gluconate, sodium gluconate, croscarmellose sodium, crospovidone, cinnamon bark powder, magnesium aluminosilicate, calcium silicate, magnesium silicate, brown rice malt, succinated gelatin, sesame oil, wheat starch, wheat germ powder, rice starch, cholesterol, safflower oil, white beeswax, dihydroxyaluminum aminoacetate, dimethylpolysiloxane (for internal use), sucrose fatty acid ester, stearyl alcohol, stearic acid, calcium stearate, polyoxyl 40 stearate, magnesium stearate, zein, sorbitan sesquioleate, cetanol, gypsum, gelatin, sorbitan fatty acid ester, hydrogenated soybean oil, soybean oil, talc, dextran 40, dextrin, sodium dehydroacetate, calcium glycerophosphate, corn syrup, corn starch, corn starch granules, tragacanth, tragacanth powder, triacetin, rapeseed oil, pearl powder, powdered juice from green barley leaves, honey, paraffin, potato starch, bitter chocolate, hydroxypropyl starch, hydroxypropyl cellulose, hydroxypropyl methylcellulose 2208, hydroxypropyl methylcellulose 2906, hydroxypropyl methylcellulose 2910, hydroxypropyl methylcellulose phthalate, castor oil, sunflower oil, phytin acid, phenacetin, glucose, hydrous glucose, pullulan, propylene glycol, propylene glycol fatty acid ester, pectin, bentonite, povidone, sodium polyacrylate, partially neutralized polyacrylic acid, polyisobutylene, polyoxyethylene (105) polyoxypropylene (5) glycol, polyoxyethylene (160) polyoxypropylene (30) glycol, polyoxyethylene hydrogenated castor oil, polyoxyethylene hydrogenated castor oil 40, polyoxyethylene hydrogenated castor oil 60, sodium polystyrene sulfonate, polysorbate 60, polysorbate 80, polyvinyl acetal diethylaminoacetate, (partially saponified) polyvinyl alcohol, polyvinyl alcohol-diethylene glycol mixture, oyster shell powder, macrogol 1500, macrogol 1540, macrogol 20000, macrogol 300, macrogol 400, macrogol 4000, macrogol 6000, multitol, yellow beeswax, myristyl alcohol, octyldodecyl myristate, methacrylic acid copolymer LD, magnesium aluminometasilicate, methyl acrylate-methacrylic acid copolymer, methyl cellulose, Japan wax, aluminum monostearate, glycerol monostearate, sodium lauryl sulfate, lauromacrogol, peanut oil, calcium monohydrogen phosphate, calcium hydrogen phosphate, calcium hydrogen phosphate particles, sodium hydrogen phosphate, potassium dihydrogen phosphate, calcium dihydrogen phosphate, sodium dihydrogen phosphate, rose oil, rosin, sodium chloride, fructose, dry chlorella, dry yeast, dried aluminum hydroxide gel, dry magnesium sulfate, baked rice cake powder, hydrogenated maltose starch syrup, hydrous silicon dioxide, hydrous amorphous silicon oxide, light silicic anhydride, light liquid paraffin, whale wax, crystalline cellulose, crystalline cellulose (particles), crystalline cellulose (fine particles), crystalline cellulose-carmellose sodium, hydrogenated oil, synthetic aluminum silicate, synthetic aluminum silicate-hydroxypropyl starch-crystalline cellulose, synthetic hydrotalcite, titanium oxide, magnesium oxide, snake oil, tartaric acid, potassium hydrogen tartrate, heavy silicic anhydride, flour, wheat germ oil, ethanol for disinfection, calcined gypsum, calcium acetate, cellulose acetate phthalate, starch syrup, magnesium aluminum hydroxide, aluminum hydroxide-magnesium carbonate-calcium carbonate coprecipitate, aluminum hydroxide-sodium hydrogencarbonate coprecipitate, aluminum hydroxide gel, magnesium hydroxide, hydrogenated vegetable oil, physiological saline solution, purified gelatin, purified shellac, purified honey, purified water, purified sucrose, purified sucrose spherical granules, soybean lecithin, unsaponified soybean oil, tricalcium phosphate, skim milk powder, simple syrup, ammonium carbonate, calcium carbonate, magnesium carbonate, medium-chain triglyceride, precipitated calcium carbonate, low-substituted sodium carboxymethyl starch, low-substituted hydroxypropyl cellulose, natural aluminum silicate, silicon dioxide, lactic acid, calcium lactate, lactose, lactose particles, concentrated glycerol, white shellac, sucrose, sucrose-starch spherical granules, half-digested starch, microcrystalline cellulose, partially pregelatinized starch, powder sugar, cellulose powder, hydrogenated maltose powder starch syrup, rice powder, citric anhydride, hydrated silicic anhydride, anhydrous calcium hydrogen phosphate, anhydrous calcium hydrogen phosphate granules, anhydrous lactose, anhydrous sodium sulfate, sterilized purified water, cotton seed oil, cotton seed oil-soybean oil mixture, medicinal charcoal, liquid paraffin, calcium sulfate, and green tea powder.

Specific examples of the solvent, solubilizer, dissolving agent, or dissolving assistant include dl-camphor, D-mannitol, D-sorbitol, D-sorbitol solution, L-aspartic acid, L-arginine, α-cyclodextrin, β-cyclodextrin, adipic acid, fennel oil, esterified corn oil, ethanol, olive oil, oleic acid, oleyl oleate, carmellose sodium, citric acid, sodium citrate, glycine, glycerol, glycerol fatty acid ester, sesame oil, safflower oil, safflower oil fatty acid, sodium salicylate, perilla oil, dibutylhydroxytoluene, sucrose fatty acid ester, stearyl alcohol, polyoxyl 40 stearate, sorbitan sesquioleate, sorbitan fatty acid ester, soybean oil, sodium dehydroacetate, corn oil, triacetin, sorbitan trioleate, tricapryline, rapeseed oil, nicotinamide, peppermint oil, hydroxypropyl cellulose, hydroxypropyl methylcellulose 2910, castor oil, phenprobamate, glucose, propylene glycol, propylene glycol fatty acid ester, povidone, polyoxyethylene (105) polyoxypropylene (5) glycol, polyoxyethylene (160) polyoxypropylene (30) glycol, polyoxyethylene hydrogenated castor oil, polyoxyethylene hydrogenated castor oil 40, polyoxyethylene hydrogenated castor oil 60, polysorbate 20, polysorbate 60, polysorbate 80, (partially saponified) polyvinyl alcohol, macrogol 1500, macrogol 200, macrogol 300, macrogol 400, macrogol 4000, macrogol 600, macrogol 6000, maleic acid, isopropyl myristate, sorbitan monooleate, sorbitan monolaurate, coconut oil, sodium lauryl sulfate, lauromacrogol, peanut oil, lidocaine, Ringer's solution, phosphoric acid, sodium hydrogen phosphate, potassium dihydrogen phosphate, sodium benzoate, benzyl benzoate, sodium chloride, magnesium chloride, dry sodium carbonate, diluted hydrochloric acid, argentine, hydrogenated oil, magnesium oxide, tartaric acid, wheat germ oil, ethanol for disinfection, water, acetic acid, cellulose acetate phthalate, sodium hydroxide, physiological saline solution, purified soybean oil, purified water, purified soybean lecithin, petroleum benzine, soybean lecithin, sodium carbonate, sodium hydrogen carbonate, medium-chain triglyceride, low-substituted hydroxypropyl cellulose, lactic acid, dark glycerol, sucrose, glacial acetic acid, anhydrous ethanol, citric anhydride, maleic anhydride, anhydrous sodium monohydrogen phosphate, sterilized purified water, cotton seed oil, and liquid paraffin.

Specific examples of the disintegrant, disintegrating assistant, dispersant, desiccant, or fluidizer include D-sorbitol, D-mannitol, adipic acid, aminoalkyl methacrylate copolymer RS, gum arabic, gum arabic powder, alginic acid, sodium alginate, propylene glycol alginate, pregelatinized starch, ethanol, ethyl cellulose, olive oil, oleic acid, carboxyvinyl polymer, sodium carboxymethyl starch, carmellose, carmellose calcium, carmellose sodium, glycyrrhiza powder, agar powder, guar gum, citric acid, calcium citrate, sodium citrate, glycerol, glycerol fatty acid ester, croscarmellose sodium, crospovidone, magnesium silicate, wheat starch, rice starch, choline phosphate, safflower oil, white beeswax, dioctyl sodium sulfosuccinate, sucrose fatty acid ester, stearic acid, calcium stearate, polyoxyl 40 stearate, magnesium stearate, sorbitan sesquioleate, gelatin, sorbitan fatty acid ester, talc, soybean oil, dextrin, corn starch, tragacanth powder, sorbitan trioleate, honey, paraffin, potato starch, hydroxyethyl cellulose, hydroxyethyl methylcellulose, hydroxypropyl starch, hydroxypropyl cellulose, hydroxypropyl methylcellulose 2910, hydroxypropyl methylcellulose 2910 mixture, castor oil, fumaric acid, monosodium fumarate, propylene glycol, propylene glycol fatty acid ester, bentonite, povidone, polyoxyethylene (105) polyoxypropylene (5) glycol, polyoxyethylene (160) polyoxypropylene (30) glycol, polyoxyethylene hydrogenated castor oil, polyoxyethylene hydrogenated castor oil 60, polyoxyethylene hydrogenated castor oil 40, polysorbate 20, polysorbate 40, polysorbate 60, polysorbate 80, polyvinyl acetal diethylaminoacetate, sodium polyphosphate, macrogol 1500, macrogol 300, macrogol 400, macrogol 4000, macrogol 6000, magnesium aluminometasilicate, sodium metaphosphate, methyl cellulose, Japan wax, sorbitan monooleate, aluminum monostearate, glycerol monostearate, sorbitan monopalmitate, sorbitan monolaurate, sodium lauryl sulfate, lauromacrogol, calcium hydrogen phosphate, calcium hydrogen phosphate granules, calcium dihydrogen phosphate, dried aluminum hydroxide gel, light aluminum oxide, light silicic anhydride, crystalline cellulose, crystalline cellulose-carmellose sodium, hydrogenated oil, hydrous silicon dioxide, synthetic aluminum silicate, titanium oxide, sodium dehydroacetate, heavy silicic anhydride, vinyl acetate resin, cellulose acetate phthalate, magnesium aluminum hydroxide, sodium hydroxide, purified shellac, purified soybean lecithin, purified sucrose, soybean lecithin, tricalcium phosphate, magnesium carbonate, sodium hydrogen carbonate, precipitated calcium carbonate, low-substituted sodium carboxymethyl starch, low-substituted hydroxypropyl cellulose, lactose, concentrated glycerol, white shellac, sucrose, partially pregelatinized starch, anhydrous ethanol, citric anhydride, anhydrous sodium citrate, anhydrous sodium pyrophosphate, and liquid paraffin.

Specific examples of the pH adjuster, isotonizing agent, or buffer include dl-malic acid, D-sorbitol, D-sorbitol solution, D-mannitol, D-tartaric acid, magnesium L-aspartate, L-arginine, L-glutamic acid, sodium L-glutamate, adipic acid, ascorbic acid, aminoethylsulfonic acid, xylytol, citric acid, calcium citrate, sodium citrate, disodium citrate, glycine, glycerol, glucono-δ-lactone, calcium gluconate, succinic acid, disodium succinate hexahydrate, nicotinamide, glucose, monosodium fumarate, propylene glycol, sodium polyphosphate, macrogol 4000, maleic acid, sodium metaphosphate, phosphoric acid, dipotassium phosphate, trisodium phosphate, sodium hydrogen phosphate, potassium dihydrogen phosphate, sodium dihydrogen phosphate, rose oil, benzoic acid, sodium benzoate, potassium chloride, calcium chloride, sodium chloride, magnesium chloride, hydrochloric acid, glucosamine hydrochloride, fructose, dry sodium sulfite, dry sodium carbonate, diluted hydrochloric acid, tartaric acid, acetic acid, sodium hydroxide, magnesium hydroxide, physiological saline solution, sodium carbonate, sodium hydrogen carbonate, lactic acid, calcium lactate, lactose, concentrated glycerol, glacial acetic acid, citric anhydride, anhydrous sodium citrate, anhydrous sodium pyrophosphate, anhydrous sodium monohydrogen phosphate, anhydrous trisodium phosphate, anhydrous dihydrogen phosphate, and sodium hydrogen sulfate.

Specific examples of the surfactant, emulsifier, or effervescent agent include D-sorbitol, ethanol, carrageenan, carboxyvinyl polymer, carmellose sodium, guar gum, glycerol, glycerol fatty acid ester, cholesterol, white beeswax, dioctyl sodium sulfosuccinate, sucrose fatty acid ester, stearyl alcohol, stearic acid, polyoxyl 40 stearate, sorbitan sesquioleate, cetanol, gelatin, sorbitan fatty acid ester, talc, sorbitan trioleate, paraffin, potato starch, hydroxypropyl cellulose, propylene glycol, propylene glycol fatty acid ester, pectin, polyoxyethylene (105) polyoxypropylene (5) glycol, polyoxyethylene (160) polyoxypropylene (30) glycol, polyoxyethylene hydrogenated castor oil, polyoxyethylene hydrogenated castor oil 40, polyoxyethylene hydrogenated castor oil 60, polyoxyl 35 castor oil, polysorbate 20, polysorbate 60, polysorbate 80, macrogol 400, octyldodecyl myristate, methyl cellulose, sorbitan monooleate, glycerol monostearate, sorbitan monopalmitate, sorbitan monolaurate, lauryl dimethylamine oxide solution, sodium lauryl sulfate, lauromacrogol, dry sodium carbonate, tartaric acid, sodium hydroxide, purified soybean lecithin, soybean lecithin, potassium carbonate, sodium hydrogen carbonate, medium-chain triglyceride, citric anhydride, cotton seed oil-soybean oil mixture, and liquid paraffin.

Specific examples of the humectant, binder, suspending agent, thickener, adhesive, adhesion enhancer, softener, or plasticizer include D-sorbitol, D-sorbitol solution, D-mannitol, RSS No. 1 crude rubber, β-captan, β-cyclodextrin, acrylic acid-octyl acrylate copolymer, 2-ethylhexyl acrylate-vinyl pyrrolidone copolymer solution, 2-ethylhexyl acrylate-2-ethylhexyl methacrylate-dodecyl methacrylate copolymer solution, acrylic ester-vinyl acetate copolymer, ethyl acrylate-methyl methacrylate copolymer dispersion liquid, acetyl glycerol fatty acid ester, acrylic acid-silk fibroin copolymer resin, methyl acrylate-2-ethylhexyl acrylate copolymer resin emulsion, acrylic resin alkanolamine solution, magnesium stearate, aminoalkyl methacrylate copolymer E, aminoalkyl methacrylate copolymer RS, aminoethylsulfonic acid, amylopectin, powdered starch syrup, gum arabic, gum arabic powder, sodium alginate, propylene glycol alginate, pregelatinized starch, ester gum, ethanol, ethyl cellulose, sodium erythorbate, phellodendron bark, olive oil, sodium oleate, kaolin, cacao butter, sodium caseinate, carrageenan, caramel, carnauba wax, carboxyvinyl polymer, carboxy vinyl polymer, carboxymethyl ethylcellulose, sodium carboxymethyl starch, carmellose, carmellose calcium, carmellose sodium, agar, agar powder, xanthan gum, benzyl acetate, guar gum, sodium citrate, glycine, glycerol, glycerol fatty acid ester, glucono-δ-lactone, magnesium silicate, magnesium aluminum silicate, copal resin, copolyvidone, sesame oil, wheat starch, rice starch, collodion, sodium chondroitin sulfate, white beeswax, dioctyl sodium sulfosuccinate, cispolyisoprene rubber, dihydroxyaluminum aminoacetate, dibutylhydroxytoluene, dimethylpolysiloxane (for internal use), dimethylpolysiloxane-silicon dioxide mixture, sucrose fatty acid ester, stearyl alcohol, stearic acid, aluminum stearate, calcium stearate, polyoxyl stearate, polyoxyl 40 stearate, styrene-isoprene-styrene block copolymer, styrene isoprene rubber, sorbitan sesquioleate, cetanol, gelatin, shellac, sorbitan fatty acid ester, solvent naphtha, soybean hydrogenated oil, talc, dammar gum, dextran 70, dextrin, terpene resin, (soluble) starch, corn starch, corn oil, tragacanth, tragacanth powder, sorbitan trioleate, rapeseed oil, high cispolyisoprene rubber [molecular weight: 47,000], honey, paraffin, potato starch, veegum neutral, hydroxyethyl cellulose, hydroxyethyl methylcellulose, hydroxypropyl starch, hydroxypropyl cellulose, hydroxypropyl methylcellulose 2208, hydroxypropyl methyl cellulose 2906, hydroxypropyl methyl cellulose 2910, hydroxypropyl methylcellulose acetate succinate, hydroxypropyl methylcellulose phthalate, vinyl pyrrolidone-vinyl acetate copolymer, piperonyl butoxide, castor oil, glucose, fumaric acid, fumaric acid-stearic acid-polyvinyl acetal diethylaminoacetate-hydroxypropyl methylcellulose 2910 mixture, sodium stearyl fumarate, pullulan, propylene glycol, propylene glycol fatty acid ester, pectin, bentonite, povidone, polyacrylic acid, sodium polyacrylate, polyacrylic aqueous solution, partially neutralized polyacrylic acid, polyisobutylene, polyoxyethylene (160) polyoxypropylene (30) glycol, polyoxyethylene hydrogenated castor oil, polyoxyethylene hydrogenated castor oil 60, polysorbate 20, polysorbate 60, polysorbate 80, polysorbate 80-polyvinyl acetal diethylaminoacetate, (entirely saponified) polyvinyl alcohol, (partially saponified) polyvinyl alcohol, polybutene, sodium polyphosphate, macrogol 1500, macrogol 20000, macrogol 300, macrogol 400, macrogol 4000, macrogol 600, macrogol 6000, maleic rosin glycerol ester, yellow beeswax, methacrylic acid-n-butyl acrylate copolymer, methacrylic acid copolymer L, methacrylic acid copolymer LD, methacrylic acid copolymer S, magnesium aluminometasilicate, sodium metaphosphate, methyl cellulose, methyl vinyl ether-maleic anhydride copolymer, Japan wax, glycerol monostearate, sodium lauryl sulfate, lauromacrogol, peanut oil, phosphoric acid, calcium hydrogen phosphate, rosin, calcium chloride, sodium chloride, magnesium chloride, hydrous lanolin, hydrolyzed gelatin powder, hydrolyzed starch-added light silicic anhydride, fructose, dried aluminum hydroxide gel, baked rice cake powder, hydrogenated starch syrup, hydrous silicon dioxide, hydrous amorphous silicon oxide, light silicic anhydride, light silicic anhydride-containing hydroxypropyl cellulose, light liquid paraffin, crystalline cellulose, crystalline cellulose-carmellose sodium, hydrogenated oil, high polyvinyl pyrrolidone, synthetic aluminum silicate, synthetic wax, titanium oxide, magnesium oxide, alicyclic saturated hydrocarbon resin, aliphatic hydrocarbon resin, sodium potassium tartrate, heavy silicic anhydride, flour, water, vinyl acetate resin, cellulose acetate phthalate, starch syrup, aluminum hydroxide gel, potassium hydroxide, sodium hydroxide, hydrogenated rosin glycerol ester, crude rubber, purified gelatin, purified shellac, purified water, purified sucrose, petroleum benzine, petroleum resin, soybean lecithin, simple syrup, calcium carbonate, magnesium carbonate, medium-chain triglyceride, precipitated calcium carbonate, low substituted hydroxypropyl cellulose, lactic acid, lactose, concentrated glycerol, white shellac, white petrolatum, sucrose, microcrystalline cellulose, partially pregelatinized starch, rice powder, anhydrous ethanol, cotton seed oil-soybean oil mixture, liquid paraffin, calcium sulfate, and sulfated castor oil potassium salt-alkylbenzenesulfonic acid mixture.

Specific examples of the coating agent, sugar-coating agent, brightener, or antifoaming agent include 2-methyl-5-vinylpyridinemethyl acrylate-methacrylic acid copolymer, dl-malic acid, D-sorbitol, D-sorbitol solution, D-mannitol, ethyl acrylate-methyl methacrylate copolymer dispersion liquid, acetyl glycerol fatty acid ester, aminoalkyl methacrylate copolymer E, aminoalkyl methacrylate copolymer RS, gum arabic, gum arabic powder, purified insect wax, ethanol, ethyl cellulose, ethyl cellulose water dispersion, octyldecyl triglyceride, Opadry OY-6950, Opadry OY-S-22829, Opadry OY-S-22835, Opadry OY-S-22961, Opadry OY-S-7135, Opadry OY-S-8471, Opadry OY-S-9607, olive oil, kaolin, cacao butter, prunella spike, hydrogenated castor wax, caramel, carnauba wax, carboxyvinyl polymer, carboxymethyl ethylcellulose, sodium carboxymethyl starch, carmellose calcium, carmellose sodium, triethyl citrate, glycerol, glycerol fatty acid ester, magnesium silicate, succinated gelatin, wheat starch, rice starch, white beeswax, dimethylaminoethyl methacrylate-methyl methacrylate copolymer, dimethylpolysiloxane (for internal use), dimethylpolysiloxane-silicon dioxide mixture, sucrose fatty acid ester, silicone resin emulsion, silicone antifoaming agent, eaglewood powder, stearyl alcohol, stearic acid, aluminum stearate, calcium stearate, polyoxyl 40 stearate, magnesium stearate, zein, sorbitan sesquioleate, cetanol, gypsum, shellac, sorbitan fatty acid ester, talc, terpene resin, (soluble) starch, corn syrup, corn oil, triacetin, sorbitan trioleate, pearl powder, honey, paraffin, potato starch, hydroxypropyl cellulose, hydroxypropyl methylcellulose 2208, hydroxypropyl methylcellulose 2906, hydroxypropyl methylcellulose 2910, hydroxypropyl methylcellulose 2910-titanium oxide-macrogol 400 mixture, hydroxypropyl methylcellulose acetate succinate, hydroxypropyl methylcellulose phthalate, piperonyl butoxide, castor oil, diethyl phthalate, butylphthalyl butylglycolate, glucose, fumaric acid-stearic acid-polyvinyl acetal diethylaminoacetate-hydroxypropyl methylcellulose 2910 mixture, pullulan, propylene glycol, bentonite, povidone, polyoxyethylene (105) polyoxypropylene (5) glycol, polyoxyethylene (160) polyoxypropylene (30) glycol, polyoxyethylene hydrogenated castor oil 40, polyoxyethylene hydrogenated castor oil 60, polysorbate 80, polyvinyl acetal diethylaminoacetate, (partially saponified) polyvinyl alcohol, macrogol 1500, macrogol 1540, macrogol 20000, macrogol 300, macrogol 35000, macrogol 400, macrogol 4000, macrogol 600, macrogol 6000, yellow beeswax, myristyl alcohol, methacrylic acid copolymer L, methacrylic acid copolymer LD, methacrylic acid copolymer S, magnesium aluminometasilicate, methyl cellulose, Japan wax, aluminum monostearate, glycerol monostearate, sorbitan monolaurate, montanic acid ester wax, sodium lauryl sulfate, lauromacrogol, calcium monohydrogen phosphate, calcium hydrogen phosphate, sodium hydrogen phosphate, calcium dihydrogen phosphoate, rosin, dry methacrylic acid copolymer LD, dried aluminum hydroxide gel, dry bleached shellac, baked rice cake powder, hydrous silicon dioxide, argentine, gold foil, silver foil, light silicic anhydride, light liquid paraffin, light silicic anhydride-containing hydroxypropyl cellulose, whale wax, crystalline cellulose, hydrogenated wax, hydrogenated oil, high glucose starch syrup, synthetic aluminum silicate; syntheticwax, titanium oxide, magnesiumoxide, flour, calcined gypsum, cellulose acetate, vinyl acetate resin, cellulose acetate phthalate, starch syrup, aluminum hydroxide gel, hydrogenated rosin glycerol ester, purified gelatin, purified shellac, purified paraffin-carnauba wax mixed wax, purified sucrose, tricalcium phosphate, simple syrup, calcium carbonate, magnesium carbonate, discolored silver foil, precipitated calcium carbonate, low-substituted hydroxypropyl cellulose, calcium lactate, lactose, concentrated glycerol, white shellac, sucrose, partially pregelatinized starch, powder sugar, hydrated silicic anhydride, phthalic anhydride, anhydrous calcium hydrogen phosphate, medicinal charcoal, liquid paraffin, and calcium sulfate.

Specific examples of the stabilizer, antioxidant, preservative, or antiseptic include adipic acid, DL-alanine, dl-camphor, D-sorbitol solution, d-borneol, D-sorbitol, D-mannitol, L-ascorbyl stearate, sodium L-ascorbate, L-aspartic acid, sodium L-aspartate, L-arginine, 1-menthol, β-cyclodextrin, ascorbic acid, aminoethylsulfonic acid, sodium alginate, propylene glycol alginate, albumin, sulfur, inositol, fennel powder, ethanol, calcium disodium edetate, sodium edetate, erythorbic acid, sodium erythorbate, cacao butter, carboxyvinyl polymer, carmellose calcium, carmellose sodium, agar, xanthan gum, xylytol, citric acid, calcium citrate, sodium citrate, glycine, glycerol, glycerol fatty acid ester, glucono-δ-lactone, calcium gluconate, cinnamon bark powder, sesame oil, sodium chondroitin sulfate, salicylic acid, sodium salicylate, phenyl salicylate, dibutylhydroxytoluene, sucrose fatty acid ester, stearic acid, aluminum stearate, polyoxyl stearate, magnesium stearate, sorbitan sesquioleate, cetanol, gelatin, sorbitan fatty acid ester, sorbic acid, potassium sorbate, talc, dextran, dextran 40, dextran 70, dehydroacetic acid, sodium dehydroacetate, disodium glycyrrhizinate, tocopherol, menthol, naphtol, nicotinamide, honey, isobutyl p-hydroxybenzoate, isopropyl p-hydroxybenzoate, ethyl p-hydroxybenzoate, butyl p-hydroxybenzoate, propyl p-hydroxybenzoate, methyl p-hydroxybenzoate, calcium pantotheate, hydroxypropyl cellulose, hydroquinone, tetrasodium pyrophosphate, sodium pyrosulfite, phenacetin, butylhydroxyanisol, glucose, fumaric acid, monosodium fumarate, povidone, partially neutralized polyacrylic acid, polyoxyethylene (160) polyoxypropylene (30) glycol, polyoxyethylene hydrogenated castor oil 60, polysorbate 20, polysorbate 60, polysorbate 80, (partially saponified) polyvinyl alcohol, potassium polyphosphate, sodium polyphosphate, macrogol 300, macrogol 400, macrogol 4000, maleic acid, malonic acid, magnesium aluminometasilicate, sodium metaphosphate, methyl cellulose, methyl hesperidin, aluminum monostearate, glycerol monostearate, eucalyptus oil, sodium lauryl sulfate, borneol powder, malic acid, phosphoric acid, sodium hydrogen phosphate, potassium dihydrogen phosphate, calcium dihydrogen phosphate, sodium sulfite, sodium hydrogen sulfite, benzoic acid, sodium benzoate, benzyl benzoate, calcium chloride, sodium chloride, magnesium chloride, hydrochloric acid, arginine hydrochloride, cysteine hydrochloride, fructose, dry sodium sulfite, dry sodium carbonate, hydrous silicon dioxide, diluted hydrochloric acid, light silicic anhydride, crystalline cellulose, hydrogenated oil, magnesium oxide, tartaric acid, acetic acid, tocopherol acetate, sodium hydroxide, magnesium hydroxide, purified gelatin, purified soybean lecithin, purified sucrose, dried aluminum hydroxide gel, soybean lecithin, unsaponified soybean oil, potassium carbonate, sodium carbonate, magnesium carbonate, potassium hydrogen carbonate, sodium hydrogen carbonate, nitrogen, natural vitamin E, lactic acid, lactose, concentrated glycerol, sucrose, microcrystalline cellulose, glacial acetic acid, propyl gallate, citric anhydride, anhydrous sodium citrate, anhydrous sodium pyrophosphate, anhydrous sodium monohydrogen phosphate, medicinal charcoal, and ovalbumin.

Specific examples of the flavor enhancer, cooling agent, perfume, or sweetener include disodium 5'-inosinate, disodium 5-guanylate, DL-alanine, dl-camphor, dl-menthol, dl-malic acid, sodium dl-malate, sodium DL-tartarate, d-camphor, D-xylose, D-sorbitol, D-sorbitol solution, d-borneol, D-mannitol, D-tartaric acid, L-aspartic acid, sodium L-aspartate, magnesium L-aspartate, L-glutamic-acid, L-arginine L-glutamate, sodium L-glutamate, L-glutamic acid hydrochloride, l-menthol, β-cyclodextrin, ascorbic acid, aspartame, gambir powder, sweet hydrangea leaf, sweet hydrangea leaf extract, sweet hydrangea leaf powder, aminoethylsulfonic acid, fennel, fennel tincture, fennel powder, fennel oil, ethanol, ethyl vanillin, erythritol, phellodendron bark powder, Japanese cherry bark extract, coptis rhizome, coptis rhizome powder, ononis root drying extract, orange, orange oil, cacao powder, caramel, carbachol, glycyrrhiza, glycyrrhiza extract, crude glycyrrhiza extract, glycyrrhiza powder, xylytol, citric acid, calcium citrate, sodium citrate, glycine, glycerol, glycyrrhizinic acid, diammonium glycyrrhizinate, dipotassium glycyrrhizinate, disodium glycyrrhizinate, monoammonium glycyrrhizinate, trisodium glycyrrhizinate, glucono-δ-lactone, chlorella extract, chlorella powder, cinnamon bark tincture, cinnamon bark powder, cinnamon bark oil, succinic acid, disodium succinate hexahydrate, monosodium succinate, seaweed powder, saccharin, saccharin sodium, saffron powder, saffron tincture, zanthoxylum peel tincture, zanthoxylum peel powder, amomum seed powder, ginger tincture, ginger powder, ginger oil, eaglewood powder, cinnamaldehyde, stearic acid, spearmint oil, swertia herb, perilla herb powder, soybean oil, jujube powder, alisma rhizome root/herb dry extract, tannic acid, clove tincture, clove oil, citrus unshiu peel tincture, red pepper, red pepper powder, red pepper tincture, bitter orange peel tincture, bitter orange peel powder, picrasma wood powder, pineapple powder flavor 51357, pineapple powder flavor 59492, honey, peppermint water, peppermint oil, vanilla powder flavor 5428632, tetrasodium pyrophosphate, wine, glucose, glucose-fructose syrup, fumaric acid, monosodium fumarate, peppermint powder, povidone, maltitol, maltitol solution, malt extract, maltose, millefolium herb dry extract, methyl cellulose, menthol powder, eucalyptus oil, borneol, borneol powder, Ringer's solution, apple juice, apple vinegar, concentrated apple juice (500%), lemon oil, rose water, rose oil, royal jelly, syrup, sodium chloride, magnesium chloride, hydrochloric acid, fructose, fructose-glucose syrup, dry yeast, hydrogenated maltose starch syrup, diluted hydrochloric acid, high glucose starch syrup, high fructose syrup, brown sugar, tartaric acid, potassium hydrogen tartrate, edible carrot powder, acetic acid, starch syrup, purified glycyrrhiza extract powder, purified honey, purified sucrose, purified sucrose spherical granules, skim milk powder, simple syrup, sodium hydrogen carbonate, medium-chain triglyceride, sodium copper chlorophyllin, lactic acid, lactose, concentrated glycerol, plum extract, sucrose, glacial acetic acid, powder sugar, hydrogenated maltose powder starch syrup, anhydrous ethanol, citric anhydride, anhydrous sodium pyrophosphate, ethyl lactate, and green tea powder.

Specific examples of the adsorbent include kaolin, carmellose calcium, hydrous silicon dioxide, magnesium silicate, light silicic anhydride, diatomite, crystalline cellulose, synthetic aluminum silicate, aluminum oxide, aluminum hydroxide, magnesium carbonate, precipitated calcium carbonate, dextrin, silicon dioxide, bentonite, magnesium aluminometasilicate, and medicinal charcoal.

Specific examples of the antifoaming agent include ethanol, glycerol fatty acid ester, dimethylpolysiloxane (for internal use), dimethylpolysiloxane-silicon dioxide mixture, sucrose fatty acid ester, silicon resin emulsion, silicon antifoaming agent, polyoxyl 40 stearate, sorbitan fatty acid ester, sorbitan trioleate, and polysorbate 80.

Specific examples of the masticatory include d-borneol.

Specific examples of the colorant include gambir tannin powder, turmelic extract solution, yellow diiron trioxide, Opaspray K-1 -24904, orange essence, brown iron oxide, carbon black, caramel, carmine, carotene solution, β-carotene, glycyrrhiza extract, gold foil, black iron oxide, light silicic anhydride, titanium oxide, diiron trioxide, food blue 1, food yellow 4, food yellow 4 aluminum lake, food yellow 5, food red 2, food red 3, food red 102, sodium hydroxide, talc, sodium copper chlorophyllin, copper chlorophyll, green barley leaf extract, d-borneol, octyldodecyl myristate, medicinal charcoal, riboflavin butyrate, riboflavin, green tea powder, and riboflavin sodium phosphate, and rose oil.

Specific examples of the soothing agent include inositol, sodium hydrogen carbonate, clove oil, glucose, propylene glycol, and lidocaine.

In the drug preparation of the present invention obtained as above, fudosteine acts on an antitussive to enhance that effect. Therefore, the preparation is markedly useful as a drug preparation with an excellent antitussive effect, particularly as a drug preparation for colds or an antitussive expectorant.

### EXAMPLES

The present invention will be described in more detail with reference to Examples and Test Examples which should not be construed as limiting the present invention.

### Test Example 1

### Antitussive effect test:

The synergetic effect of using fudosteine and an antitussive compound in combination was examined according to the following method. The results are shown in Table 1.

### (Method)

Five male Hartley guinea pigs for one group, each having a weight of 300-400 g, were used as test animals. According to the method of Miyata et al. (Arch. Int. Pharmacodyn. Ther., vol. 304, p. 277, 1990), the guinea pigs were exposed to SO₂ gas using an exposure apparatus for two hours a day in eight consecutive days to cause respiratory tract inflammation. The samples of which the components and dose are shown in Table 1 were orally administered before exposure to SO₂ gas once a day in eight consecutive days since the start date of exposure. The guinea pigs were confined in a chamber without being anesthetized. On the date following the last SO₂ exposure date, capsaicin (10⁻⁶ mol/l) was sprayed over the chamber for two minutes using a ultrasonic nebulizer (TUR-3200, Nihon Kohden Corporation) to cause cough in the guinea pigs. The reaction was observed for two minutes during spraying and 13 minutes following the spraying (15 minutes in total). The frequency of cough during this period was counted.

### (Results)

**Table 1**

| Test group | Frequency of cough |
|---|---|
| Control group | 22.4 ± 0.8 |
| Fudosteine 30 mg/kg | 22.6 ± 1.1 |
| Dextromethorphan 150 mg/kg | 13.6 ± 1.0 ^{**} |
| Dextromethorphan 300 mg/kg | 10.2 ± 0.6 ^{**},^{$} |
| Fudosteine (30 mg/kg) + Dextromethorphan (150 mg/kg) | 7.2 ± 0.7 ^{**},^{##},^{$$} |
| Fudosteine (30 mg/kg) + Dextromethorphan (300 mg/kg) | 5.2 ± 0.4 ^{**},^{##},⁺⁺ |

| | |
|---|---|
| **: p < 0.01, for control group | |
| ##: p < 0.01, for fudosteine 30 mg/kg group | |
| $: p < 0.05, for dextromethorphan 150 mg/kg group | |
| $$: p < 0.01, for dextromethorphan 150 mg/kg group | |
| ++: p < 0.01, for dextromethorphan 300 mg/kg group | |

These results show that fudosteine did not exhibit an antitussive effect when administered alone. In contrast, dextromethorphan exhibited an antitussive effect when administered alone. Its antitussive effect was enhanced by administration in combination with fudosteine. The dose response was also observed. The synergetic effect on the antitussive effects of using the two medicinal components in combination was exhibited. As is clear from these results, the antitussive effect of the antitussive was enhanced by using fudosteine in combination.

### Example 1

### Tablet preparation:

Fudosteine (1,200 g), dextromethorphan hydrobromide (60 g), corn starch (38 g), talc (1 g), and magnesium stearate (1 g) were made into a powder for tableting by a conventional method. The powder was tableted using a rotary tableting machine to prepare a tablet preparation in which each tablet had a diameter of 8.5 mm and a weight of 250 mg.

### Example 2

### Liquid preparation:

Fudosteine (8 g), dextromethorphan hydrobromide (400 mg) , erythritol (20 g), caramel (100 mg), sodium benzoate (70 mg), and flavor (100 mg) were dissolved in purified water. An appropriate amount of phosphoric acid was added to the mixture to adjust the pH, followed by adding purified water to the mixture to make the total amount 100 ml, to prepare a liquid preparation.

### Example 3

### Tablet preparation:

Fudosteine (120 g), dihydrocodeine phosphate (3 g), dl-methylephedrine hydrochloride (5 g), chlorpheniramine maleate (0.8 g), anhydrous caffeine (9 g), potato starch (159.8 g), talc (1.2 g), and magnesium stearate (1.2 g) were made into a powder for tableting by a conventional method. The powder was tableted using a rotary tableting machine to prepare a tablet preparation in which each tablet had a diameter of 8.5 mm and a weight of 250 mg.

### Example 4

### Granule preparation:

Fudosteine (120 g), dihydrocodeine phosphate (3 g), lyzozyme hydrochloride (3.3 g), chlorpheniramine maleate (7.5 g) , corn starch (89.7 g) , and trehalose (83.25 g) were made into a granule preparation by a conventional method.

### Example 5

### Liquid preparation:

Fudosteine (12.8 g), codein phosphate (0.2 g), dl-methylephedrine hydrochloride (0.3 g), lyzozyme hydrochloride (0.24 g, titer), chlorpheniraminemaleate (48 mg), anhydrous caffeine (0.24 g), senega powder extract (60 g), erythritol (60 g), and flavor (0.4 g) were dissolved in purified water. An appropriate amount of phosphoric acid was added to the mixture to adjust the pH, followed by adding purified water to the mixture to make the total amount 240 ml, to prepare a liquid preparation.

## Claims

1. A drug preparation comprising fudosteine and a medicine possessing an antitussive effect.

2. The drug preparation according to claim 1, wherein the preparation is a preparation to be orally administered.

3. The drug preparation according to claim 1 or 2, wherein the preparation is an antitussive expectorant.

4. The drug preparation according to any one of claims 1-3, further comprising one or more medicines selected from the group consisting of a bronchodilator, antihistamine, analeptic, galenical, bactericide, and antacid or mucosal protective agent.
